# EUROPEAN PATENT SPECIFICATION

(11) **EP 1 267 954 B1**
(45) Date of publication and mention of the grant of the patent: **09.08.2006**
(21) Application number: 01910963.6
(22) Date of filing: 16.02.2001
(51) Int. Cl.: A61L 27/38, A61L 27/54

(54) **MEDICAL DEVICES SUITABLE FOR GENE THERAPY REGIMENS**
MEDIZINISCHE VORRICHTUNGEN FÜR GENTHERAPIEVERFAHREN
DISPOSITIFS MEDICAUX CONVENANT A DES SCHEMAS POSOLOGIQUES DE THERAPIE GENIQUE

(30) Priority: 04.04.2000 US 542935
(43) Date of publication of application: 02.01.2003
(73) Proprietor: Boston Scientific Limited, St. Michael (BB)
(72) Inventor: PALASIS, Maria, Wellesley, MA 02481 (US)
(74) Representative: Altenburg, Udo
(86) International application number: PCT/US2001/005324
(87) International publication number: WO 2001/074413

(56) References cited:
- WO-A-00/32255
- WO-A-95/07691
- WO-A-95/24929
- WO-A-99/00071
- US-A- 5 628 785
- US-A- 5 698 531
- LUIGI BIANCONE ET AL: 'Development of Inflammatory Angiogenesis by Local Stimulation of Fas In Vivo' J. EXP. MED. vol. 186, no. 1, 01 July 1997, pages 147 - 152

## Description

### Field of the Invention

The present invention relates to a medical device and a method for preparing a medical device for controlled delivery and long term expression of one or more products and therapeutic agents within the body of a patient. In particular, this invention relates to gene or cell therapies wherein the genetic material is coated on a surface of an insertable or implantable medical device and is delivered to a target location in the patient's body.

### I. Background of the Invention

Gene therapy provides an attractive approach for combating many intractable cardiovascular diseases. Prior art gene therapy regimens suffer from several limitations rendering application ofthis therapy unsuccessful in a clinical setting. Short term expression of therapeutic gene products, low levels of gene expression, and lack of site-specificity are among several factors that are responsible for the failure of this system of therapy *in vivo.* The lack of adequate integration into the host genome, or the rejection of the transgene or carrier vector by the immune response, are among several factors that account for the short duration and low level expression of gene products.

Advances in interventional radiology and innovative designs in balloon angioplasty and stents have raised the therapeutic potential of gene and cell therapy regimens. Long term delivery of drugs has been achieved through sustained and localized delivery of drugs *in vivo*. One potential drawback to conventional localized drug administration is the uncontrolled manner by which the drug or drug solution is released from the delivery device. It is often necessary to control and/or lengthen the time period over which the drug is released. For example, it might be advantageous to lengthen the release time from hours to days or even weeks. Exceptionally long release times, as long as several months, are often desired, for example, where the drug is released from an implanted device such as a stent.

The therapeutic use of conventional drug delivery devices is further limited by problems associated with the increased trauma to the vessel wall, rapid washout of drug, use of therapeutic agents having a single mechanism of action, and the like. For a more comprehensive review of this subject see, for example: "Effect of controlled adventitial heparin delivery on smooth muscle proliferation following endothelial injury", Edelman E., *et al.,* Proc. Natl. Acad. Sci. U.S.A., 87:3773, 1990; "Localized release of perivascular heparin inhibits intimal proliferation after endothelial injury without systemic anticoagulation", Okada T., *et al.,* Neurosurgery, 25:892, 1989; "Iontophoretic transmyocardial drug delivery: A novel approach to antiarrhythmic drug therapy", Avitall B*.*, *et al.,* CIRC., 85:1582, 1992; "Direct intraarterial wall injection of microparticles *via* a catheter: A potential drug delivery strategy following angioplasty," Wilinsky R., *et al.,* Amer. Heart J., 122: 1136, 1991; and "Local anticoagulation without systemic effect using a polymer hepatin delivery system", Okada T., *et al.,* Stroke, 19:1470, 1988.

One way to achieve site-specificity and long term expression of a therapeutic gene product *in vivo* is the incorporation of genetic material, in the form of a naked or plasmid DNA, into a device coating. Transformation efficiency of naked or plasmid DNA, however, is very low relative to DNA delivered by a lipid carrier or by viral vectors. Viral vectors present a very efficient means of introducing foreign genes into cells. Unfortunately, viral vectors are usually very labile and thus not able to withstand the coating process without losing activity. Thus, the incorporation of viral particles into a device coating is likely not to be feasible in its present form.

Several reports in scientific literature indicate that recombinant viral vectors facilitate efficient transfer of foreign genes into somatic tissues, including vascular endothelial and smooth muscle cells. Early therapy-directed strategies relied on gene transfer by placement of a device containing transformed cells within the body of a patient. The concept of direct gene transfer to the target cell population, obviating the need for cell culture, has prompted the development of catheter based gene delivery systems. Although several prototype systems have demonstrated *in vivo* delivery of viral vectors to a vascular wall, resulting in expression of foreign genes, currently available systems are limited by the quantity and infectivity of virus delivered, traumatic injury to the vessel wall during delivery, and interruption of blood flow during delivery.

Adenoassociated virus (AAV) is a viral particle with unique characteristics. It is solvent and heat stabile so that it may withstand the processing conditions required to incorporate the virus into a device coating. Furthermore, it can be stored at room temperature for several months without losing activity. The use of vectors derived from adenoassociated virus vectors for transferring genes *in vitro* and *in vivo* has been described (see, in particular, U.S. Pat. Nos. 4,797,368; 5,139,941; and 5,851,521). These patents describe various adenoassociated virus derived constructs in which the replicase (rep) or cap genes are deleted and replaced by a gene of interest. WO 990071 refers to a method for coating stents comprising a biocompatible structure carrying a genetic material comrising a first therapeutic agent comprising a vector containing a first polynucleotide wherein said vector is an adenoassociated virus vector and a second therapeutic agent, i.e., antimicrobial agent, anticoagulant agent, antimitotic agent.

A drawback to adenoassociated virus delivery of genes is that the onset of protein expression is delayed by approximately two weeks post introduction into the cell. Treatment ofproliferative diseases such as restenosis will likely require immediate protein expression in order to effect the proliferative response which generally peaks about three days post device implantation and usually subsides by two weeks.

This invention, as disclosed and described herein, overcomes the above-mentioned limitations of prior art gene delivery and drug delivery systems. The device of this invention facilitates delivery ofa polynucleotide sequence of interest in a controlled and stable manner *in vivo.* The mechanical problems associated with the prior art insertable or implantable medical devices have been overcome by the invention described herein via the use of a polymer coating which is capable of providing adequate tensile strength, permitting control of porosity and hydration capacity of the device. A new gene therapy regimen is disclosed that overcomes prior art problems regarding stability during coating process, site-specificity, and stability of expression of a polynucleotide sequence *in vivo.*

These characteristics, and several others as disclosed herein, render the medical device of this invention desirable for a variety of therapeutic methods, including gene and cell therapies.

### II. Summary of the Invention

In one aspect of the invention, there is provided a medical device having a biocompatible structure carrying a genetic material. The biocompatible structure comprises a biocompatible polymeric coating that coats at least a portion of the structure and carries a genetic material. The genetic material comprises: (a) a first therapeutic agent comprising a vector containing a first polynucleotide that establishes a gene expression sufficient to produce a therapeutically sufficient amount of one or more products encoded by said first polynucleotide; and (b) a second therapeutic agent comprising a non-genetic therapeutic agent, wherein said non-genetic therapeutic agent is an angiogenic agent.

The genetically coated medical device of the invention preferably comprises a stent, catheter or combination thereof. In a more preferred embodiment of this invention, the medical device or structure of the invention comprises a stent composed of a biocompatible material, including biostable or biodegradable materials. More preferably, the stent is a metallic stent. The stent is in any shape and made of any biocompatible material suitable for keeping all or part of a tubular or spiral apparatus in an opened, expanded, coiled or tubular form during storage and/or following implantation. Suitable biocompatible materials for a stent include, but are not limited to, stainless steel, nickel, silver, platinum, gold, titanium, iridium, tungsten, Ni-Ti Alloys (*i.e*., Nitinol), inconel, poly-1-lactic acid (PLLA) / poly ε-caprolactone (PCL) blends, and the like.

The medical device of the invention is provided a method of inhibiting or treating restenosis in a subject, preferably a mammal, by administering to the subject, at a predetermined site in the body. The site of administration is preferably a site of mechanical injury to an arterial wall produced by treatment of an atherosclerotic lesion by angioplasty.

In yet anther aspect of the invention, there is provided a method of preparing a medical device for controlled delivery of a genetic material to a subject. The method comprises: (A) applying a polymer coating to at least a portion of a medical device; (B) applying a genetic material to said polymer coating to obtain a genetically coated medical device, said genetic material comprising: (a) a first therapeutic agent comprising a vector containing a first polynucleotide that establishes a gene expression sufficient to produce a therapeutically sufficient amount of an angiogenic agent and (b) a non-genetic therapeutic agent that is an angiogenic agent. Steps (A) and (B) are not limited to orderly steps and the method encompasses application of B prior to or concurrent with A.

### III. Detailed Description Of The Invention

As used herein, the following terms are defined as follows:
***"Naked polynucleotide",*** as used herein includes any nucleic acid molecule, not incorporated into a viral, plasmid, or other non-plasmid polynucleotide carrier.
***"Genetic material",*** as used herein, includes any biologically active material used in the cell therapy or gene therapy of this invention, whether, *in vivo, ex vivo* or *in vitro* cultures, including; bacterial, viral, prokaryotic or eukaryotic cell cultures in a transformed or non-transformed states; native or foreign polynucleotides, recombinant or natural polynucleotides, including reporter genes, marker genes, regulatory sequences, antisense molecules, and the like. Additionally, the genetic material may include non-genetic agents in combination with aforementioned compounds and compositions.
***"Product",*** as used herein, includes any protein, peptide, polypeptide, polynucleotide, in sense or antisense orientation, including DNA, RNA, DNA/RNA hybrids. The terms protein, peptide, and polypeptide are used interchangeably herein.
***"Therapeutic agent"**,* as used herein, includes anycompound, composition or small molecule that induces a beneficial biological or a medical reaction *in vitro, ex vivo,* or *in vivo.* The therapeutic agents are, for example, any protein, peptide, polypeptide, polynucleotides, small molecules, antibiotics, non-genetic agents, therapeutic polymers, cells or a combination thereof.
***"Medical device"**,* as used herein, includes any implantable or insertable medical device, whether in part or in whole.
***"Stent"**,* as used herein, includes any tubular or helical device used to maintain or support a bodily orifice or cavity, including grafts.
***"Balloon catheter"**,* as used herein, includes a catheter having a balloon or multiple balloons that can be inflated or deflated without removal of the catheter after insertion into the body.
***"Therapeutically sufficient amount**"*, as described herein means, an effective amount of an agent or product that is determined based on the nature and the chemical composition of the particular agent or product used.

This invention describes a medical device and a method to deliver a genetic material within the vasculature or ducts of a patient. The method and device of the invention, as disclosed herein, provide control over the delivery, target location, time of exposure, duration and amount of a one or more products and therapeutic agents within the body of a patient. Local expression of genes is particularly desirable in therapies involving the use of a chemotherapeutic agent, specifically when therapy is directed to a particular organ or site and requires non-systemic exposure of that site within the body. The gene delivery system, according to this invention, in combination with the use of devices such as stents or intravenous catheters, provides an effective device and system of gene delivery *both in vivo* and *ex vivo.*

The medical device of this invention is a device adapted for introduction into the body, for example, into the esophagus, trachea, colon, intestines, biliary tract, urinary tract, vascular system, ureters, urethra, bronchi, pancreatic duct system, gut, nasolacrimal duct, sinus cavities, eye, fallopian tubes, and the like.

Specifically, as disclosed herein, the medical device, according to this invention, is any insertable or implantable medical device. Examples of the medical device of the invention, include without limitation, catheters, stents, needle injection catheter, blood clot filters, vascular grafts, stent grafts, biliary stents, colonic stents, bronchial / pulmonary stents, esophageal stents, urethral stents, aneurysm filling coils and other coiled coil devices, trans myocardial revascularization ("TMR") devices, precutaneous myocardial revascularization ("PMR"), hypodermic needles, soft tissue clips, holding devices, and the like.

The medical device of the invention is delivered to and /or implanted at target locations by known techniques. Delivery is optionally performed with a sheath that covers the coated medical device to help inhibit the release of the genetic material prior to reaching a target location.

While the structure included in the medical device may be configured in a variety of ways, the structure is preferably configured as a stent composed of a biocompatible material, including biostable or biodegradable materials. More preferably, the stent is a metallic stent. The stent is in any shape and made of any biocompatible material suitable for keeping all or part of a tubular or coiled coil apparatus in an opened, expanded, coiled or tubular form during storage and/or following implantation. Suitable biocompatible materials for a stent include, but are not limited to, stainless steel, nickel, silver, platinum, gold, titanium, iridium, tungsten, Ni-Ti Alloys (*i.e.,* Nitinol), inconel, poly-1-lactic acid (PLLA) / poly ε-caprolactone (PCL) blends, or the like.

In a preferred embodiment of this invention, there is provided on the outer surface of a medical device, such as stent, a biodegradable, resorbable polymer. The stent, according to the invention disclosed herein, provides adequate tensile strength to function as a scaffolding device, permits control of porosity and hydration capacity, and, when desired, degrades into products that are nontoxic to the genetic material impregnated thereon or to the cells of the vascular wall of the patient.

Included within the scope of this invention are stents that comprise a microporous tube or coil spring fabricated, for example, from aliphatic polyester blends using a floration-precipitation or manual casting/winding techniques.

Another preferred medical device for use with the present invention is a coated stent that is used in conjunction with a balloon catheter. According to this embodiment of the invention, a stent attached to a balloon catheter is introduced into an artery. Inflation of the balloon expands the stent and presses it slightly into the wall of the treated artery. When the balloon is deflated, the stent is left behind to bolster the arterial wall. More preferably, the stent is a balloon-expansible stent or a self-expanding stent, *i.e.,* of the type formed with superelastic materials such as Nitinol, as described in U.S. Patent No. 5,954,706.

The invention also encompasses a catheter for delivering genetic material at a desired location within the body or within the wall of body lumen. The catheter has preferably an expandable portion mounted on a catheter shaft, the expandable portion preferably being expandable to a controlled pressure, i.e., to fill the cross-section of a body lumen and press against the wall of the body lumen.

The device, or at least one portion of a surface of the device that comes into contact with a target site, is coated with a polymeric coating. Any suitable surface of the medical device may be coated with a polymeric coating. The surfaces to be coated may comprise, for example, wells, holes, grooves, slots, flat surfaces, upper or inner surfaces, and the like contained within or on the medical device. It is not necessary that an entire surface be coated, rather, merely a portion of a surface may be coated. For example, if an expandable catheter is used, at least a portion of the exterior surface of the expandable portion is coated with a polymeric coating.

In another preferred embodiment of this invention, there is provided a polymer coating that protects and covers a surface of the medical device and prevents release of the genetic material from the coating during transvascular or transductal delivery until it is delivered to the target location within the body. The polymer coating preferably regulates the release kinetics of the genetic material from the medical device.

As polymeric coating, any medically acceptable natural or synthetic polymer is used. According to one embodiment of this invention, the polymer comprises, for example, water soluble, thermally degradable, or biodegradable polymers. Examples of the polymer of this invention include, but are not limited to, polycaprolactone, polyorthoesters, polylactic acids, poly-1-lactic acid /poly ε-caprolactone blends, polyglycolic acids, carbowax, gelatin, chitosan, polyvinyl alcohol, polyethylene oxide, polyethylene glycol, albumin, pluronic gel F-127, collagen, alginates, cellulosic compounds, polyvinyl pyrrolidone, or a combination thereof.

Also encompassed withing the scope of the invention are biostable polymers with porosity sufficient to allow release of therapeutics. Genetic material, including transformed cells do not need to be released but may stay trapped in a biostable matrix release protein.

The thickness of the polymeric coating is preferably from 1 to 1000 µm, more preferably from 10 to 100 µm, and most preferably from 5 to 50 µm, per total layers. Layers of polymer coating, form 1 to 40 layers, each layer having a thickness of from 1 to 10 µm/ layer of coating, are applied to the appropriate surface(s) of the medical device.

When the coated surface(s)of the medical device comes into contact with the patient's blood, or other tissues and organs within the body, the genetic material is substantially released therefrom. Depending on the choice of the polymeric coating, the site of release, or the particular gene or cell therapy strategy intended, the release of the genetic material is either immediate or follows a controlled lag time of about 1 to several minutes (for example, 1, 5,10, 20, 30, 60, or 90 minutes) to allow the medical device to reach the target site.

The genetic material, according to one embodiment of this invention, includes one or more polynucleotides, carried by a vector in combination with a carrier. The vector and the carrier are each part of the first and the second therapeutic agent, respectively. The vector, carrier, or both, are used to transform, transfect, or transduce eukaryotic or prokaryotic cells, which cells are then applied onto or impregnated into the same or a different layer of coating. The terms transform, transduce, and transfect are used interchangeably herein. In a preferred embodiment of this invention, the genetic material comprises transfected cells.

Transformed cells deliver products and therapeutic agents of interest at the transplant site. The delivery media is formulated as needed to maintain cell function and viability. Cells used in cell therapy are derived, for example, from the patient, or from a different source, including mammalian or non-mammalian sources, and from a variety of tissues and organs, including for example, bone marrow, blood, or liver among others. In general, cells are of human origin (autologous or allogenic) or of animal origin (xenogenic). This invention encompasses the use of cells in a wide range of developmental and growth conditions. For example, the use of differentiated cells, undifferentiated cells, stem cells, or progenitor cells, are all contemplated within the scope of this invention. Undifferentiated cells , if desired, are chemically or physically induced to differentiate into a desired cell type.

The vector, according to the invention described herein, comprises a recombinant vector. Preferably, the vector is a viral or plasmid vector, more preferably the vector is a viral vector that is replication deficient, thermostable, site specific and delayed expression viral vector. Most preferably, the viral vector is stable, withstands coating processes, and has low immunogenicity. An example of the preferred vector of this invention is the adenoassociated viral vector.

In a preferred embodiment of the invention, the vector of the present invention is a delayed expression vector that delays the expression of the first polynucleotide generally from 1 to 3 weeks after administration to a patient. The expression of products and the stability of such expression within the body of a patient are ultimately influenced by factors, such as, the particular site of administration, *i.e.,* organ, tissue or cell types, within a patient's body.

The carrier of this invention comprises, for example, a viral vector, non-viral vector, non-plasmid vector or naked nucleic acid. The carrier is preferably an early expression carrier that provides transient expression of the second polynucleotide of interest which it carries. The early expression carrier generally results in expression of the second polynucleotide within 1 to 2 days or sooner after administration of the medical device within the body of a patient. This transient expression may continue for several weeks in target cells.

According to one embodiment of the invention, the first polynucleotide is carried by a delayed expression vector and the second polynucleotide is carried by an early expression vector. The first polynucleotide integrates into the genome of cells at or around the target site and establishes a delayed, and stable expression with a level sufficient to produce a therapeutically effective amount of one or more products in these cells. An example of a delayed expression is an expression delayed from about two days to about three weeks. The sufficient frequency of expression is an expression that is induced in from 5% to 90% of cells exposed to the vector. On average from 20% to 80% of the cells exposed to the vector express the product encoded by the first polynucleotide.

A preferred non-plasmid vector of this invention includes, for example, liposomes, lipofectin, lipoplexes, polyplexes, dextrans, starburst dendrimer conjugates, polybrene dimethyl sulfoxide, protamine sulfate, antibody conjugates, polylysine conjugates, gramacidin S, artificial conjugates, viral envelopes, viral-like particles, nano or micro particles, polyvinyl pyrrolidone, SP1017,or a combination thereof.

It should be understood that the design of the vector and the carrier, as discussed above, may depend on factors, such as, the choice of the host cell to be transformed and/or the type of the products desired to be expressed. Moreover, the vector copy number, the ability to control that copy number and the expression of any other products encoded by the first or the second polynucleotide of this invention, such as antibiotic markers, or antisense molecules should also be considered.

Non-limiting examples of viral vectors or vectors derived from viral sources include adenoviral vectors, herpes simplex vectors, papilloma vectors, adenoassociated viral vectors, retroviral vectors, alpha virus vectors , lentivirus vectors, and the like. As described above, the viral vector of this invention is preferably replication defective and, thus, unable to replicate autonomously in the target cell. In general, the genome of the replication defective viruses, which is used within the scope of the present invention, lacks at least the region necessary for the replication of the virus in the infected cell. These regions can either, be eliminated (in whole or in part), rendered non-functional or substituted by other sequences, in particular by the sequence of the gene of interest. The replication defective virus may retain the sequences of its genome necessary for encapsidating the viral particles.

As described above, a preferred vector of this invention is a viral vector derived from an adenovirus or adenoassociated virus (AAV). Adenoassociated viruses are DNA viruses of relatively short size which integrate, in a stable and site-specific manner, into the genome of cells which they infect. These viruses are able to infect a wide spectrum of cells without inducing any effects on cellular growth, morphology or differentiation. Furthermore, they do not appear to be involved in human pathologies and demonstrate low immunogenicity.

Two general types of adenoassociated virus vectors are used. In the first type, the capsid gene is deleted and foreign DNA (about 2 kb) is inserted at the deletion site. This type of vector retains the replicase gene and can integrate at a specific site in the host genome. The second type of vector simply contains one or more AAV terminal repeats (plus a few additional bases) on both ends of the foreign DNA. In some cases such a vector can integrate and transform with >50% frequency, but the incidence of site specificity may be reduced.

Various serotypes of adenovirus having varying structures and properties exist. Of these serotypes, preference is given, within the scope of the present invention, to type 2 or type 5 human adenoviruses (Ad 2 or Ad 5) or adenoviruses of animal origin, but other adenoassociated subtypes, such as, subtypes 1, 3, 4, and 6-42 may also be used. Those adenoviruses of animal origin which can be used within the scope of the present invention include adenoviruses of canine, bovine, or murine sources. Preferably, the adenovirus of animal origin is a canine adenoassociated virus type 2 (CAV2) or it is of a mixed human and canine origin.

Included within the scope of the present invention is the use of adenoviruses sharing the major structural features of Ad:Pac-beta Galactosidase (beta-Gal) gene. That is, the invention includes the use of a replication deficient E1-, E3-deleted adenovirus comprising, for example, Ela enhancer/packaging signals at the 3' end. The viruses have in the E1 deletion site a non-adenoviral encoding sequence operably linked to a promoter. The non-adenoviral encoding sequence can be a marker gene (such as the nuclear localizing beta-Gal gene, the luciferase gene, and the like). The encoding sequence can also be a gene encoding a therapeutically active molecule (examples of which are set forth above). The promoter to which the encoding sequence is operably linked is, for example, a strong viral promoter, such as the CMV promoter/enhancer or the respiratory syncytial virus (RSV) promoter, a tissue specific promoter, or an inducible promoter (*i.e.*, the metallothionine promoter).

The vector and carrier carry the first and the second polynucleotide, respectively. The first and the second polynucloetide are the same or a different polynucleotides according to this invention and are in any molecularly acceptable form. Molecularly acceptable forms include, for example, DNA, ribozymes, anti-sense DNA or RNA, naked DNA, cDNA, RNA, DNA/RNA hybrid, genomic DNA, and the like.

The first or second polynucleotide, or both encodes one or more products. Products comprise, for example, proteins, peptides, polypeptides, or polynucleotide molecules in sense or antisense orientation. A product is understood to be any translational, post-translationally modified, or non-translational product of a polynucleotide regardless of size and form. Products, include as a primary example, those products that can compensate for a defect or deficiency in an animal, or those that act through toxic effects to limit or remove harmful cells from the body.

The first therapeutic agent of this invention comprises genetic materials whereas the second therapeutic agent of the invention may comprise either genetic or non-genetic materials. The non-genetic material comprises any molecule or compound that induces a beneficial biological or medical reaction *in vitro, ex vivo,* or *in vivo.* An example of a beneficial biological reaction is a reaction that stimulates and increases the chance of incorporation of the genetic material into the cells.

Included within the scope of the therapeutic agents of the invention are those agents that enhance gene transfer and integration into cells and tissues. These agents include compounds such as, for example, polybrene, poly-L-lysine, dextran sulfate, polyvinyl pyrrolidone, SP 1017 (Supratek Pharma), other polycationic substances, dexamethasone, substances which can increase the permeability of tissues such as detergents and lipids, for example, NP-40, and SDS that increase the permeability of tissues. Further examples of the therapeutic agents of the invention include immunosuppressants, such as, cyclosporin, dexamethasone, and antibodies against cytokines, such as, TNF-alpha, among others.

Non-limiting examples of products and therapeutic agents of the invention include: anti-thrombogenic agents such as heparin, heparin derivatives, urokinase, and PPACK (dextrophenylalanine proline arginine chloromethylketone); tissue plasminogen activator; erythropoietin; antioxidants; angiogenic and anti-angiogenic agents and factors; agents blocking smooth muscle cell proliferation such as rapamycin, angiopeptin, monoclonal antibodies capable of blocking smooth muscle cell proliferation; anti-inflammatory agents such as estrogen; calcium entry blockers; antineoplastic/antiproliferative/ anti-mitotic agents such as , angiostatin and thymidine kinase inhibitors; nitrofurantoin; anesthetic agents such as lidocaine, bupivacaine, and ropivacaine; nitric oxide synthase (NOS); anti-coagulants such as D-Phe-Pro-Arg, an RGD peptide-containing compound; antithrombin compounds; platelet receptor antagonists; anti-thrombin antibodies; anti-platelet receptor antibodies; prostaglandin inhibitors; platelet inhibitors; vascular cell growth promotors; growth factor receptor antagonists; transcriptional activators and translational promotors; vascular cell growth inhibitors such as growth factor inhibitors; growth factor receptor antagonists; transcriptional repressors; translational repressors; replication inhibitors; inhibitory antibodies; antibodies directed against growth factors; bifunctional molecules consisting of a growth factor and a cytotoxin, bifunctional molecules consisting of an antibody and a cytotoxin; cholesterol-lowering agents; vasodilating agents; agents which interfere with endogenous vasoactive mechanisms; and survival genes which protect against cell death, such as anti-apoptotic Bcl-2 family factors.

According to one embodiment of the invention, the first, or the second polynucleotide, or both encode angiogenic and anti-angiogenic agents such as acidic and basic fibroblast growth factors, vascular endothelial growth factor, hif-1, epidermal growth factor, transforming growth factor α and β, platelet-derived endothelial growth factor, platelet-derived growth factor, tumor necrosis factor α, hepatocyte growth factor and insulin like growth factor, AKT, cell cycle inhibitors including CDK inhibitors, retinoblastoma, p53, p16, p27, anti-restenosis agents, thymidine kinase ("TK"), FAS ligand, hKIS, heme oxygenase, nitric oxides, and combinations thereof.

Still other products, which can be encoded by the first or the second polynucleotide sequences, or both include monocyte chemoattractant protein ("MCP-1 "), and the family of bone morphogenic proteins ("BMP's".) The known proteins include BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect on BMP expression can be provided. Such molecules include any of the "hedgehog" proteins, or the nucleic acids encoding them.

In general, the first or the second polynucleotide, or both may each be operably linked to at least one transcriptional regulatory sequence in a manner that allows expression of the polynucleotide sequence. Regulatory sequences are art-recognized and are selected to direct gene expression. Accordingly, the term transcriptional regulatory sequence includes promoters, enhancers and other expression control elements. Such regulatory sequences are described in Goeddel; Gene Expression Technology: Methods in Enzymology 185, Academic Press, San Diego, Calif. (1990).

The regulatory sequences, within the scope of the invention can include, for example, sequences which are naturally responsible for expressing a gene, provided that these sequences are also capable of functioning in the infected cell. The sequences can also be sequences of a different origin (responsible for expressing or regulating the expression of different proteins or even synthetic proteins). In particular, the sequences can be sequences of eukaryotic or viral genes or sequences derived therefrom, which sequences, for example, stimulate or repress transcription of a gene in a specific or non-specific manner or in an inducible or non-inducible manner. As an example, they can be promoter sequences derived from the host genome or from the genome of a virus, in particular the promoters of the adenoviral E1A and MLP genes, CMV or LTR-RSVpromoter.

Other regulatory sequences within the scope of this invention include eukaryotic promoters comprising ubiquitous promoters (HPRT, vimentin, actin, tubulin, etc.), intermediate filament promoters (desmin, neurofilaments, keratin, GFAP, etc.); therapeutic gene promoters (MDR type, CFTR, factor VIII, etc.); tissue-specific promoters (actin promoter in smooth muscle cells); promoters which are preferentially activated in dividing cells, or promoters which respond to a stimulus (steroid hormone receptor, retinoic acid receptor, etc.). The early and late promoters of SV40; adenovirus or cytomegalovirus immediate early promoter; lac system; trp system; T7 promoter, whose expression is directed by T7 RNA polymerase; major operator and promoter regions of phage lambda; control regions for viral coat protein; promoter for 3-phosphoglycerate kinase or other glycolytic enzymes; promoters of acid phosphatase, i.e., Pho5; promoters of the yeast alpha-mating factors; polyhedron promoter of the baculovirus system and other sequences known to control the expression of genes of prokaryotic or eukaryotic cells or their viruses, and various combinations thereof, are included within the scope of this invention.

In a preferred embodiment of the invention, the first polynucleotide, the second polynucleotide, or both are linked, upstream of the coding sequence, to a signal sequence which directs the synthesized products into the secretory pathways of the target cell. While this signal sequence can be a natural signal sequence, it can also be any other functional signal sequence (that of the gene for thymidine kinase, for example), or an artificial signal sequence.

The amount of genetic material carried on the medical device is an effective expression-inducing amount. As used herein, the term "effective expression-inducing amount" means that amount of the genetic material that effectuates expression of a product encoded by one or more of the polynucleotide sequence(s) of interest. Means for determining an effective expression-inducing amount of a nucleic acid sequence is well known in the art.

As described above, the first and the second polynucleotide of this invention are carried by the vector and carrier, respectively. According to their particular structure and design, vector and carrier molecules affect expression and integration of the polynucleotides within the body of the recipient. The vector and carrier system of this invention ensure continued expression of a product *in vivo.*

According to another embodiment of the invention, the second polynucleotide is the same as the first polynucleotide, and is carried by an early expression vector that either integrates into the genome of the target cells or enters a transient expression route expressing the product(s) transiently in target cells. Transient expression usually lasts about one to about four weeks after administration of the medical device to a patient.

For example, the first therapeutic agent comprises a polynucleotide sequence encoding a cell cycle inhibitor gene, such as RB, or a promoter of endothelialization, such as vascular endothelial growth factor (VEGF). These genes are carried by a delayed expression vector. The second therapeutic agent comprises a polynucleotide sequence encoding a cytotoxic gene such as FasL, which is carried by an early expression carrier. Therefore, the release kinetics and the duration of release of the first and the second therapeutic agents are controlled.

In general, the release kinetics of the genetic material are governed by several factors, including for example, the structure of the medical device, the structure, porosity, and thickness of the polymeric coating, the nature of the genetic material (*i.e.,* transformed cells, polynucleotide carriers, etc..), the target site of therapy, and so on. A fine balance of these parameters provides a controlled, and stable release of the genetic material from the device coating into or proximal to a target site within the body of a patient.

Expressed products, according to the invention, are in general useful in any application for treating, preventing, or otherwise affecting the course of a disease or tissue or organ dysfunction. For example, products are used to inhibit angiogenesis, prevent or treat restenosis, treat a cardiomyopathy, heart failure, or other dysfunction of the heart; treat cystic fibrosis or other dysfunction of the lung; treat or inhibit malignant or non-malignant cell proliferation; or to treat inducing nerve, blood vessel or tissue regeneration in a particular tissue or organ.

A preferred embodiment of this invention is to provide treatment of vascular thrombosis and angioplasty restenosis, particularly coronary vascular thrombosis, and angioplasty restenosis, thereby to decrease incidence of vessel rethrombosis and restenosis, unstable angina, myocardial infarction and sudden death. The medical device and method of this invention can be used to treat patients having severe complications resulting from thrombus. Specific examples include patients with acute myocardial infarction (AMI) and patients that have failed PTCA (percutaneous transluminal coronary angioplasty) and have abrupt thrombotic closure of the targeted artery.

Organs and tissues that are treated by the methods of the present invention include any mammalian tissue or organ, whether injected *or* implanted and whether *in vivo* or *ex vivo.* Non-limiting examples include the heart, lung, brain, liver, skeletal muscle, smooth muscle, kidney, bladder, intestines, stomach, pancreas, ovary, prostate, cartilage and bone. Other organ systems susceptible to treatment include any organ system in which material flows through the organ from a source, so that a factor can be administered in a coating. Exemplary organs include lymph nodes, the bile duct, the urinary tract, the lungs, the space occupied by the cerebro-spinal fluid, and the like. Examples of other tissues which can be treated in this manner include the stomach and intestines, where growth factors help accelerate repair of ulceration, repair of external ulceration of skin, and general wound repair.

It will be appreciated that because transformation of appropriate target cells *in vivo* represents a first step in gene therapy, choice of the particular gene delivery system will depend on such factors as the phenotype of the intended target and the route of administration. Furthermore, it will be recognized that the particular polynucleotide construct provided for *in vivo* transformation is also useful for *in vitro* transformation of cells, such as for use in the *ex vivo or in situ* tissue culture systems.

Having now fully described the invention, the same would be more readily understood by reference to specific examples which are provided by way of illustration, and not intended to be limiting of the invention, unless herein specified.

### Example 1. Intravascular Local Gene Transfer Mediated by Coated Metallic Stent

A replication-defective recombinant adenovirus carrying Lac Z reporter gene for nuclear-specific β-galactosidase(Ad- β gal) is used in this study. Coating of the stent is performed by immersing a metallic stent in gelatin solution containing crosslinker for 10 seconds, then air drying. The thickness of the coating is controlled to about 5-10 µm. The coated stent is mounted on a 4.0 or 3.0 mm PTCA (percutaneous transluminal coronary angioplasty) balloon and submersed into Ad- β gal viral stock (2 x10¹⁰ pfu/ml) for 3 min then implanted into carotid arteries in a rat. Coating of the stent and implantation is repeated in a significant number of rats.

The animals are sacrificed at 7,14 and 21days after implantation. β-galactosidase expression is assessed by X-Gal staining. The expression oftransgene can be detected in all animals. In vessels with denuded endothelium gene expression is found in sub-intima, media and adventitia. This study shows the feasibility and efficiency of adenovirus-mediated gene transfer to localized arterial wall by a protein-coated metallic stent.

### Example 2. Fabrication of Resorbable Microporous Intravascular Stents or Gene And Cell Therapy Applications

Resorbable, microporous endoluminal stents are prepared from poly-1-lactic acid (PLLA)/poly ε-caprolactone (PCL) blends. Both helical and tube stent designs are obtained by solvent casting and flotation-precipitation fabrication techniques. A range ofPLLA/PCL blend ratios and process variables are employed to investigate their influence on mechanical properties, porosity, and degradation rate of the stent. Polymer blends with higher PLLA proportions exhibit higher elastic moduli and ultimate tensile strength, lower elongation, porosity, and degradation rates than polymer blends with higher PCL content. Stents with suitable mechanical properties for deployment and support of the vessel wall are obtained. Poly(ethylene oxide) is incorporated into these devices using an acid swelling technique, opening the pore structure and improving the hydrophilic character, thereby enabling the uptake of recombinant adenoviral vectors. The 50:50 PLLA/PCL blended stents are impregnated with recombinant adenovirus (ADCM β Gal, encoding a nuclear localizing variant of *Escherichia coli* β-galactosidase). Cultured CV-1 cells incubated with stents impregnated with the recombinant virus express nuclear localized β-galactosidase activity, confirming that absorbed virus is released from the matrix in an infectious form, with kinetics suggesting that genetically enhanced endovascular devices of this design are feasible.

### Example 3. Biological Activity of the Released DNA

The biological activity of adenovirus DNA, released from the surface of a coated medical device, is investigated by transfecting HEK 293 cells *in vitro.* The result indicates that the released DNA is biologically active and has a high transfection efficiency.

### Example 4. Preparation of Replication Deficient Adenovirus Vectors

Using standard recombinant DNA techniques, replication deficient adenoviral virions are produced that express a gene encoding vascular endothelial growth factor (VEGF). Successful recombination of the plasmid and the adenoviral DNA results in the generation of a full adenoviral genome (minus, for example, an E1 or E3 deletions), with the polynucleotide sequence of VEGF cloned into the E1and/or E3 deleted regions. The adenovirus can include one or more endonuclease cleavage sites, *i.e.,* restriction sites unique to the adenovirus. Various sites can be used. Preferably, however, the sequence recognized by the endonuclease is at least 6 bases in length, more preferably, at least 8, downstream from the cleavage site(s). An endonuclease cleavage site can also be present at the E3-deletion site. The recombinant virions are replication deficient because they lack essential E1 adenoviral gene sequences. However, the HEK293 cell line is stably transfected with the E1 gene, thus supplying its essential function in trans. The replication deficient recombinant adenovirus is thus replication efficient in 293 cells, producing cell lysis and virion production. The virions that are produced do not produce lytic infections in other cell types, but they can express VEGF.

### Example 5. Preparation of Replication Deficient Adenoassociated Virus Vectors

**The replication defective recombinant adenoassociated virus vector (AAV) is** prepared by cotransfecting a plasmid containing a polynucleotide sequence of interest flanked by two AAV inverted terminal repeat (ITR) regions, and a plasmid carrying the AAV encapsidation genes (rep and cap genes), into a cell line which is infected with a human helper virus (for example an adenovirus). The AAV terminal repeats (plus a few more bases) are present on both ends of the foreign DNA. The AAV recombinants which are produced are then purified by standard techniques. The replication defective recombinant adenoassociated vectors are also made by deleting the capsid gene and substituting the foreign DNA (about 2 kb). The vectors integrate into the genome of the recipient cells and transform these cells with >50% frequency.

### Example 6. Pre Clinical/ Clinical Applications of the Adenoassociated Virus Gene Therapy

**Experiment 1.** A nitric oxide synthase (NOS)-containing adenoassociated vector is used following coronary angioplasty to prevent restenosis. Administration is effected via a catheter (*i.e.,* a balloon-tipped catheter) or, for example, via a stent. The NOS-SMC (smooth muscle cell) containing adenoassociated virus is used to promote reendothelialization and reduce proliferation of the injured segment, and thereby prevent restenosis.

**Experiment 2.** A nitric oxide synthase (NOS)-containing adenoassociated vector is used to treat atherosclerotic arteries. Endothelial dysfunction is an early event in atherosclerosis. Introduction of a NOS-containing adenovirus at a lesion site prevents progression to complex lesions, and promotes healing once developed. Administration is effected as described above (*i.e*, by catheter or by stent).

**Experiment 3.** A nitric oxide synthase (NOS)-containing adenoassociated vector is used in anti-cancer therapy. NOS is used to promote cell death. Accordingly, the NOS-adenoviruses vector is delivered to well vascularized tumors to effect the generation of cytotoxic levels of NOS. Administration can be effected by direct injection or implantation into the tumor tissue or, for example, by intravascular injection. The particular isoform used is mNOS.

### Example 7. Expression of Two Products Encoded by Two Polynucleotide Sequences of Interest Carried By Two Expression Vectors

The replication defective recombinant adenoassociated virus vector (AAV) is prepared by cotransfecting a plasmid containing vascular endothelial growth factor (VEGF) flanked by two AAV inverted terminal repeat (ITR) regions, and a plasmid carrying the AAV encapsidation genes (rep and cap genes), into a cell line which is infected with a human helper virus (for example an adenovirus). The AAV terminal repeats (plus a few more bases) are present on both ends of the foreign DNA. The AAV recombinants which are produced are then purified by standard techniques. The replication defective recombinant adenoassociated vectors are also made by deleting the capsid gene and substituting the foreign DNA (about 2 kb) with a polynucleotide sequence encoding the whole or a portion of a cell cycle inhibitor gene or a promoter of endothelialization, such as vascular endothelial growth factor (VEGF) is cloned into the replication-defective AAV vector produced above. The AAV recombinant vector thus produced is used as the delayed expression vector. The second sequence of interest is a polynucleotide sequence encoding FAS ligand. This sequence is cloned into an adenovirus derived vector under the direction of the early promoter of SV40. Both the AAV recombinant vector and the adenovirus derived vector are applied onto, or impregnated into, one or several layers of a coating on the surface of a stent. The stent is administered to a patient following coronary angioplasty to prevent restenosis. The VEGF containing AAV recombinant vector is used to promote reendothelialization of the injured segment, and thereby prevent restenosis.

Both the early expression vector and the delayed expression vectors are effective in this therapy. The delayed expression vector integrates into the genome of the recipient cells and transform these cells with >50% frequency. The early expression vector transiently expresses the product of FAS L after about 1 or 2 days of delivery to target cells. The transient expression of FAS L continues about 1 to 4 weeks in the patient's body.

## Claims

1. A medical device comprising:
a biocompatible structure carrying a genetic material, said biocompatible structure comprising a polymeric coating that coats at least a portion of said structure, said genetic material comprising:
(a) a first therapeutic agent comprising a vector containing a first polynucleotide that establishes a gene expression sufficient to produce a therapeutically sufficient amount of one or more products encoded by said first polynucleotide, wherein said first polynucleotide encodes an angiogenic agent; and
(b) a second therapeutic agent comprising a non-genetic therapeutic agent, wherein said non-genetic therapeutic agent is an angiogenic agent.

2. The medical device of claim 1, wherein said vector is an adenoassociated virus vector.

3. The medical device of claim 1, wherein said vector comprises a viral vector.

4. The medical device of claim 3, wherein said vector is thermostable, replication-deficient, non-immunogenic, or a combination thereof.

5. The medical device of claim 1, wherein said expression is achieved in 20 % to 80 % of cells exposed to said genetic material.

6. The medical device of claim 1, wherein said polymeric coating comprises polyurethane, silicone, EVA, poly-1-lactic acid/poly e-caprolactone blends, or a combination thereof.

7. The medical device of claim 1, wherein said polymer coating is from 1 to 40 layers having a thickness of from 1 to 10 µm/layer of coating.

8. The medical device of claim 1, wherein said structure is a stent.

9. The medical device of claim 8, wherein said stent is a metallic stent.

10. The medical device of claim 8, wherein said stent is a coil spring made from aliphatic polyester blends.

11. The medical device of claim 8, wherein said stent is a microporous tube made from aliphatic polyester blends.

12. The medical device of claim 1, wherein said first therapeutic agent and said second therapeutic agent are applied onto or impregnated into a same layer of said polymer coating.

13. The medical device of claim 1, wherein said vector is site specific.

14. The medical device of claim 1, wherein said vector contains regulatory sequences.

15. A method of preparing a medical device for controlled delivery of a genetic material comprising:
(A) applying a polymer coating to at least a portion of a medical device;
(B) applying a genetic material to said polymer coating to obtain a genetically coated medical device, said genetic material comprising; (a) a first therapeutic agent comprising a vector containing a first polynucleotide that establishes a gene expression sufficient to produce a therapeutically sufficient amount of an angiogenic agent; and (b) a non-genetic therapeutic agent that is an angiogenic agent.

16. The method of claim 15, wherein said vector is adenoassociated virus vector.

17. The method of claim 15, wherein said vector comprises a viral vector.

18. The method of claim 17, wherein said viral vector is thermostable, replication-deficient, non-immunogenic, or a combination thereof.

19. The method of claim 15, wherein said expression is achieved in 20 % to 80 % of cells exposed to said genetic material.

20. The method of claim 15, wherein said vector is a delayed expression vector.

21. The method of claim 20, wherein said delayed expression is an expression delayed from two days to 3 weeks after administration in vivo.

22. The method of claim 15, wherein said coating comprises polyurethane, silicone, EVA, poly-1-lactic acid/poly e.caprolactone blends, or a combination thereof.

23. The method of claim 15, wherein said polymer coating is from about 1 to about 40 layers having a thickness of from 1 to 10 µm/layer of coating.

24. The method of claim 15, wherein said medical device is a stent.

25. The method of claim 24, wherein said stent is a coil spring made from aliphatic polyester blends.

26. The method of claim 24, wherein said stent is a microporous tube made from aliphatic polyester blends.

27. The method of claim 24, wherein said stent is a metallic stent.

28. The method of claim 15, wherein said first therapeutic agent and said non-genetic therapeutic agent are applied onto or impregnated into a same layer of said polymer coating.

29. The method of claim 15, wherein said non-genetic therapeutic agent is a protein.

30. The method of claim 15, wherein said non-genetic therapeutic agent is a small molecule.

31. The method of claim 15, wherein said non-genetic therapeutic agent is a non-protein based agent.

32. The method of claim 15, wherein said vector comprises liposomes, lipofectin, lipoplexes, polyplexes, dextrans, starburst dendrimer conjugates, polybrene dimethyl sulfoxide, protarnine sulfate, antibody conjugates, polylysine conjugates, gramacidin S, artificial conjugates, viral envelopes, viral-like particles, nano or micro particles, or a combination thereof.

33. The method of claim 15, wherein said vector is site specific.

34. The method of claim 15, wherein said vector contains regulatory sequences.

## Patentansprüche

1. Medizinische Vorrichtung umfassend:
Eine ein genetisches Material tragende biokompatible Struktur, wobei die biokompatible Struktur eine Polymerbeschichtung umfasst, die wenigstens einen Teil der Struktur bedeckt, wobei das genetische Material umfasst:
(a) einen ersten therapeutischen Wirkstoff umfassend einen Vektor, der ein erstes Polynucleotid enthält, der eine Genexpression liefert, die ausreichend ist zum Herstellen einer therapeutisch ausreichenden Menge eines oder mehrerer Produkte, die von dem ersten Polynucleotid kodiert werden, wobei das erste Polynucleotid einen angiogenetischen Wirkstoff kodiert; und
(b) einen zweiten therapeutischen Wirkstoff, der einen nicht-genetischen therapeutischen Wirkstoff umfasst, wobei der nicht-genetische therapeutische Wirkstoff ein angiogenetischer Wirkstoff ist.

2. Medizinische Vorrichtung gemäß Anspruch 1, wobei der Vektor ein adeno-assoziierter viraler Vektor ist.

3. Medizinische Vorrichtung gemäß Anspruch 1, wobei der Vektor einen viralen Vektor umfasst.

4. Medizinische Vorrichtung gemäß Anspruch 3, wobei der Vektor thermostabil, replikations-defizient, nicht-immunogen, oder eine Kombination davon ist.

5. Medizinische Vorrichtung gemäß Anspruch 1, wobei die Expression in 20% bis 80% der dem genetischen Material ausgesetzten Zellen erreicht wird.

6. Medizinische Vorrichtung gemäß Anspruch 1, wobei die Polymerbeschichtung Polyurethan, Silikon, EVA, Poly-L-Milchsäure/Poly-e-caprolacton-Gemische oder eine Kombination davon umfasst.

7. Medizinische Vorrichtung gemäß Anspruch 1, wobei die Polymerbeschichtung 1 bis 40 Schichten mit einer Dicke von 1 bis 10 µm/Beschichtungsschicht aufweist.

8. Medizinische Vorrichtung gemäß Anspruch 1, wobei die Struktur ein Stent ist.

9. Medizinische Vorrichtung gemäß Anspruch 8, wobei der Stent ein metallischer Stent ist.

10. Medizinische Vorrichtung gemäß Anspruch 8, wobei der Stent eine aus aliphatischen Polyester-Gemischen hergestellte Spiralfeder ist.

11. Medizinische Vorrichtung gemäß Anspruch 8, wobei der Stent eine aus aliphatischen Polyester-Gemischen hergestellte mikroporöse Röhre ist.

12. Medizinische Vorrichtung gemäß Anspruch 1, wobei der erste therapeutische Wirkstoff und der zweite therapeutische Wirkstoff in eine gleiche Schicht der Polymerbeschichtung aufgebracht oder darin imprägniert werden.

13. Medizinische Vorrichtung gemäß Anspruch 1, wobei der Vektor ortsspezifisch ist.

14. Medizinische Vorrichtung gemäß Anspruch 1, wobei der Vektor regulatorische Sequenzen enthält.

15. Verfahren zum Herstellen einer medizinischen Vorrichtung zur gesteuerten Abgabe eines genetischen Materials, wobei das Verfahren umfasst:
(A) Aufbringen einer Polymerbeschichtung auf wenigstens einen Teil einer medizinischen Vorrichtung;
(B) Aufbringen eines genetischen Materials auf die Polymerbeschichtung unter Erhalt einer genetisch beschichteten medizinischen Vorrichtung, wobei das genetische Material umfasst; (a) einen ersten therapeutischen Wirkstoff umfassend einen ein erstes Polynukleotid enthaltenden Vektor, der eine Genexpression liefert, die ausreichend ist zum Herstellen einer therapeutisch ausreichenden Menge eines angiogenetischen Wirkstoffs; und (b) einen nicht-genetischen therapeutischen Wirkstoff, der ein angiogenetischer Wirkstoff ist.

16. Verfahren gemäß Anspruch 15, wobei der Vektor ein adeno-assoziierter viraler Vektor ist.

17. Verfahren gemäß Anspruch 15, wobei der Vektor einen viralen Vektor umfasst.

18. Verfahren gemäß Anspruch 17, wobei der virale Vektor thermostabil, replikations-defizient, nicht-immunogen, oder eine Kombination davon ist.

19. Verfahren gemäß Anspruch 15, wobei die Expression in 20% bis 80% der dem genetischen Material ausgesetzten Zellen erreicht wird.

20. Verfahren gemäß Anspruch 15, wobei der Vektor ein Vektor vom verzögerten Expressionstyp ist.

21. Verfahren gemäß Anspruch 20, wobei die verzögerte Expression eine Expression ist, die von zwei Tagen bis drei Wochen nach Verabreichung in vivo verzögert ist.

22. Verfahren gemäß Anspruch 15, wobei die Beschichtung Polyurethan, Silikon, EVA, Poly-L-Milchsäure/Poly-e-caprolaton-Gemische, oder eine Kombination davon umfasst.

23. Verfahren gemäß Anspruch 15, wobei die Polymerbeschichtung ungefähr 1 bis ungefähr 40 Schichten mit einer Dicke von 1 bis 10 µm/Beschichtungsschicht aufweist.

24. Verfahren gemäß Anspruch 15, wobei die medizinische Vorrichtung ein Stent ist.

25. Verfahren gemäß Anspruch 24, wobei der Stent eine aus aliphatischen Polyester-Gemischen hergestellte Spiralfeder ist.

26. Verfahren gemäß Anspruch 24, wobei der Stent eine aus aliphatischen Polyester-Gemischen hergestellte mikroporöse Röhre ist.

27. Verfahren gemäß Anspruch 24, wobei der Stent ein metallischer Stent ist.

28. Verfahren gemäß Anspruch 15, wobei der erste therapeutische Wirkstoff und der nicht-genetische therapeutische Wirkstoff auf eine gleiche Schicht der Beschichtung aufgebracht oder darin imprägniert werden.

29. Verfahren gemäß Anspruch 15, wobei der nicht-genetische therapeutische Wirkstoff ein Protein ist.

30. Verfahren gemäß Anspruch 15, wobei der nicht-genetische therapeutische Wirkstoff ein kleines Molekül ist.

31. Verfahren gemäß Anspruch 15, wobei der nicht-genetische therapeutische Wirkstoff ein nicht-Protein-basierter Wirkstoff ist.

32. Verfahren gemäß Anspruch 15, wobei der Vektor Liposomen, Lipofektin, Lipoplexe, Polyplexe, Dextrane, Starburst Dendrimer-Konjugate, Polybren-dimethylsulfoxid, Protaminsulfat, Antikörper-Konjugate, Polylysin-Konjugate, Gramazidin S, künstliche Konjugate, virale Hüllen, virus-artige Partikel, Nano- oder Mikro-Partikel oder eine Kombination davon umfasst.

33. Verfahren gemäß Anspruch 15, wobei der Vektor ortspezifisch ist.

34. Verfahren gemäß Anspruch 15, wobei der Vektor regulatorische Sequenzen enthält.

## Revendications

1. Un dispositif médical comprenant :
une structure biocompatible portant un matériau génétique, ladite structure biocompatible comprenant un revêtement de polymère qui enrobe au moins une partie de ladite structure, ledit matériau génétique comprenant :
(a) un premier agent thérapeutique comprenant un vecteur renfermant un premier polynucléotide qui établit une expression de gène suffisante pour produire une quantité thérapeutiquement suffisante d'un ou plusieurs produits codés par ledit premier polynucléotide, dans lequel ledit premier polynucléotide code un agent angiogénique, et
(b) un deuxième agent thérapeutique comprenant un agent thérapeutique non génétique, dans lequel ledit agent thérapeutique non génétique est un agent angiogénique.

2. Le dispositif médical selon la revendication 1, dans lequel ledit vecteur est un vecteur virus adéno-associé.

3. Le dispositif médical selon la revendication 1, dans lequel ledit vecteur comprend un vecteur viral.

4. Le dispositif médical selon la revendication 3, dans.lequel ledit vecteur est thermostable, déficient en réplication, non immunogénique ou une combinaison de ceux-ci.

5. Le dispositif médical selon la revendication 1, dans lequel ladite expression est obtenue en exposant 20 % à 80 % des cellules audit matériau génétique.

6. Le dispositif médical selon la revendication 1, dans lequel ledit revêtement polymère comprend du polyuréthane, de la silicone, du EVA, des mélanges d'acide poly-1-lactique/poly e-caprolactone ou une combinaison de ceux-ci.

7. Le dispositif médical selon la revendication 1, dans lequel ledit revêtement de polymère est constitué de 1 à 40 couches ayant une épaisseur de 1 à 10 µm/couche d'enrobage.

8. Le dispositif médical selon la revendication 1, dans lequel ladite structure est un écarteur.

9. Le dispositif médical selon la revendication 8, dans lequel ledit écarteur est un écarteur métallique.

10. Le dispositif médical selon la revendication 8, dans lequel ledit écarteur est un ressort bobiné constitué de mélanges de polyesters aliphatiques.

11. Le dispositif médical selon la revendication 8, dans lequel ledit écarteur est un tube microporeux constitué à partir de mélanges de polyesters aliphatiques.

12. Le dispositif médical selon la revendication 1, dans lequel ledit premier agent thérapeutique et ledit deuxième agent thérapeutique sont appliqués ou imprégnés dans une même couche dudit revêtement de polymère.

13. Le dispositif médical selon la revendication 1, dans lequel ledit vecteur est spécifique au site.

14. Le dispositif médical selon la revendication 1, dans lequel ledit vecteur renferme des séquences régulatoires.

15. Procédé pour préparer un dispositif médical pour la fourniture contrôlée d'un matériau génétique comprenant :
(A) l'application d'un revêtement de polymère à au moins une partie d'un dispositif médical ;
(B) l'application d'un matériau génétique audit revêtement de polymère pour obtenir un dispositif médical enrobé génétiquement, ledit matériau génétique comprenant (a) un premier agent thérapeutique comprenant un vecteur renfermant un premier polynucléotide qui établit une expression de gène suffisante pour produire une quantité thérapeutiquement suffisante d'agent angiogénique et (b) un agent thérapeutique non génétique qui est un agent angiogénique.

16. Le procédé selon la revendication 15, dans lequel ledit vecteur est un vecteur virus adéno-associé.

17. Le procédé selon la revendication 15, dans lequel ledit vecteur comprend un vecteur viral.

18. Le procédé selon la revendication 17, dans lequel ledit vecteur viral est thermostable, déficient en réplication, non immunogénique ou une combinaison de ceux-ci.

19. Le procédé selon la revendication 15, dans lequel ladite expression est obtenue dans 20 % à 80 % des cellules exposées audit matériau génétique.

20. Le procédé selon la revendication 15, dans lequel ledit vecteur est un vecteur à expression retardée.

21. Le procédé selon la revendication 20, dans lequel ladite expression retardée est une expression retardée de deux jours à trois semaines après l'administration in vivo.

22. Le procédé selon la revendication 15, dans lequel ledit revêtement comprend du polyuréthane, de la silicone, EVA, des mélanges d'acide poly-1-lactique/poly e-caprolactone ou une combinaison de ceux-ci.

23. Le procédé selon la revendication 15, dans lequel ledit revêtement de polymère est constitué d'environ 1 à environ 40 couches ayant une épaisseur de 1 à 10 µm/couche d'enrobage.

24. Le procédé selon la revendication 15, dans lequel ledit dispositif médical est un écarteur.

25. Le procédé selon la revendication 24, dans lequel ledit écarteur est un ressort bobiné constitué de mélanges de polyesters aliphatiques.

26. Le procédé selon la revendication 24, dans lequel ledit écarteur est un tube microporeux constitué de mélanges de polyesters aliphatiques.

27. Le procédé selon la revendication 24, dans lequel ledit écarteur est un écarteur métallique.

28. Le procédé selon la revendication 15, dans lequel ledit premier agent thérapeutique et ledit agent thérapeutique non génétique sont appliqués ou imprégnés dans une même couche dudit revêtement de polymère.

29. Le procédé selon la revendication 15, dans lequel ledit agent thérapeutique non génétique est une protéine.

30. Le procédé selon la revendication 15, dans lequel ledit agent thérapeutique non génétique est une petite molécule.

31. Le procédé selon la revendication 15, dans lequel ledit agent thérapeutique non génétique est un agent de base sans protéine.

32. Le procédé selon la revendication 15, dans lequel ledit vecteur comprend des liposomes, de la lipofectine, des lipoplexes, des polyplexes, des dextranes, des conjugués dendrimères du starburst, du diméthyl sulfoxyde de ploybrène, du sulfate de protamine, des conjugués d'anticorps, des conjugués de polylysine, de la gramacidine S, des conjugués artificiels, des enveloppes virales, des particules analogues aux virus, des nano ou microparticules, ou une combinaison de ceux-ci.

33. Le procédé selon la revendication 15, dans lequel ledit vecteur est spécifique au site.

34. Le procédé selon la revendication 15, dans lequel ledit vecteur renferme des séquences régulatoires.
